# EUROPÄISCHE PATENTSCHRIFT

(11) **EP 1 173 541 B1**
(45) Veröffentlichungstag und Bekanntmachung des Hinweises auf die Patenterteilung: **01.09.2004**
(21) Anmeldenummer: 00927100.8
(22) Anmeldetag: 28.04.2000
(51) Int. Cl.: C12M 1/34

(54) **VORRICHTUNG ZUR DURCHFÜHRUNG VON UNTERSUCHUNGEN AN ZELLKULTUREN**
DEVICE FOR PERFORMING ANALYSES ON CELL-CULTURES
DISPOSITIF POUR EFFECTUER DES ANALYSES SUR DES CULTURES CELLULAIRES

(30) Priorität: 06.05.1999 DE 19920811
(43) Veröffentlichungstag der Anmeldung: 23.01.2002
(73) Patentinhaber: Micronas GmbH, 79108 Freiburg (DE)
(72) Erfinder: LEHMANN, Mirko, D-79102 Freiburg (DE); BRISCHWEIN, Martin, D-97424 Schweinfurt (DE); BAUMANN, Werner, D-77815 Bühl (DE); EHRET, Ralf, D-79291 Merdingen (DE); FREUND, Ingo, D-79235 Vogtsburg-Oberrotweil (DE); WOLF, Bernhard, D-79252 Stegen (DE)
(74) Vertreter: Patentanwälte Westphal, Mussgnug & Partner
(86) Internationale Anmeldenummer: PCT/EP2000/003860
(87) Internationale Veröffentlichungsnummer: WO 2000/071669

(56) Entgegenhaltungen:
- EP-A- 0 608 153
- EP-A- 0 818 540
- WO-A-90/04645
- FR-A- 2 690 926
- US-A- 4 125 436
- US-A- 5 494 044

## Beschreibung

Die Erfindung bezieht sich auf eine Vorrichtung zur Durchführung von Untersuchungen an lebenden Zellen, Zellkulturen und dergleichen, insbesondere zur Detektion der Stoffwechselaktivität der Zellen, die sich in einem flüssigen Medium befinden, wobei die Vorrichtung wenigstens eine Aufnahme für flüssige Medium und die Zellkultur aufweist und wobei ein oder mehrere Meßein richtungen und/oder Sensoren für Messungen an der Zellkultur vorgesehen sind und wobei ein bewegbarer Trennkörper vorgesehen ist, der einen Reaktionsraum begrenzt.

Bei einer solchen, bekannten Vorrichtung wird den Zellen in einer bestimmten zeitlichen Abfolge frisches Kulturmedium oder ein in diesem Kulturmedium gelöster Wirkstoff zugeführt beziehungsweise verbrauchtes Medium aus dem Zellkulturbereich entfernt. Eine häufige Regeneration des Kulturmediums, beispielsweise durch ein geeignetes Fluidiksystem, erzeugt ein weitgehend konstantes physiologisches Milieu. Leicht zersetzliche Wirkstoffe, die dem Nährmedium zugesetzt sind, können ebenfalls leichter regeneriert werden. Das zugeführte Medium und der Zellkulturbereich selbst müssen vor Kontamination durch Mikroorganismen und vor übermäßiger Verdunstung geschützt sein. Dies sind wichtige Voraussetzungen für die sensitive Messung zellulärer Reaktionen.

Gemeinsam ist allen Zellkulturbereichen eine Fläche am Boden für die Zellaufbewahrung/Zellwachstum sowie eine Wandung, die einen Trog für die Aufnahme des Kulturmediums bildet. Das Kulturmedium muß in regelmäßigen Zeitabständen regeneriert werden, da sich Abfallprodukte des Zellstoffwechsels akkumulieren, Nährstoffe verbraucht werden und biologisch aktive Substanzen ihre Aktivität im Laufe der Zeit einbüßen.

Aus der EP 0 394 406 ist bereits eine Vorrichtung für das Fluid-Handling von Zellkulturen in Kombination mit Sensoren bekannt. Diese Vorrichtung weist eine dicht abgeschlossene, kleinvolumige Perfusionskammer auf, in der die Zellen kultiviert werden und die gleichzeitig mit einem Sensor bestückt ist. Die Kammer besitzt einen Zu- und einen Abflußkanal. Angetrieben von einer Flüssigkeitspumpe, fließt das Kulturmedium durch die Perfusionskammer. In periodisch aufeinanderfolgenden Intervallen folgen Phasen mit Perfusion und Phasen ohne Perfusion aufeinander. Die Phasen mit Perfusion dienen der Regeneration des Kulturmediums, die Phasen ohne Perfusion dem Meßvorgang, d.h. der direkten Verfolgung der extra-zellulären Ansäuerung in der Perfusionskammer.

Nachteilig ist jedoch, daß ein hoher apparativer Aufwand vorhanden ist, da eine Flüssigkeits-Antriebspumpe, Ventile zur Steuerung der Flüssigkeitsströme sowie Schlauchführungen nötig sind.
Nachteilig ist weiterhin, daß eine gewisse Anfälligkeit für die Bildung von Luftblasen in der Perfusionskammer vorhanden ist, die nur unter vergleichsweise hohem Aufwand auszuschließen sind. Dazu sind Anlagen zur Teil-Entgasung des Mediums erforderlich, die den Zellkulturen vorgeschaltet werden müssen. Dies erhöht insgesamt den Aufwand. Schließlich ist ein relativ hoher Arbeitsaufwand erforderlich, um eine luftblasenfreie und luft- bzw. flüssigkeitsdichte Montage des Systems zu erzielen. Besonders nachteilig wirkt sich dies aus, wenn eine Vielzahl paralleler Proben zu testen sind, was in der Praxis die Regel ist.

Aus der FR-A-2 690 926 ist ein Bioreaktor bekannt, der einen Behälter aufweist, in dem Feststoffpartikel mit einer Flüssigkeit in Kontakt gebracht werden können. In dem Behälter ist ein Kolben angeordnet, der verstellbar ist, so daß das Volumen des Reaktors verändert werden kann.

Aus der WO-A-90/04645 ist eine Mikro-Durchflußkammer bekannt, in der sich Zellen zur Untersuchung sowie ein Sensor befinden. Mittels eines Zulaufs und eines Ablaufs steht die Durchflußkammer mit der äußeren Umgebung in Verbindung.

Aus der EP-A-0 608 153 ist eine Dosiervorrichtung mit einer Reaktionskammer bekannt. Innerhalb der Reaktionskammer ist ein Kolben geführt, der in mehrere, unterschiedliche Positionen verstellbar ist. Seitlich an der Wand der Reaktionskammer sind in unterschiedlichen Abständen zum Boden der Reaktionskammer Leitungen angeschlossen, die je nach Stellung des Kolbens freigegeben oder verdeckt werden.

Die drei vorgenannten Druckschriften befassen sich mit Vorrichtungen, die vergleichsweise aufwendig und kompliziert im Aufbau sind. Eine Untersuchung an Zellen durch Einstellung der unterschiedlichen, geometrischen Umgebungsbedingungen von Zellkulturen ist damit nicht möglich.

Aufgabe der vorliegenden Erfindung ist die Schaffung einer vergleichsweise einfachen Anordnung zur Regeneration von Zellkulturlösungen und die Schaffung kleiner Meßvolumina. Dabei soll auch die Möglichkeit gegeben sein, die geometrischen Umgebungsbedingungen der Zellkultur anpassen zu können.

Zur Lösung dieser Aufgabe wird vorgeschlagen, daß der Trennkörper in Meßposition nahe oberhalb einer auf einem Boden der Aufnahme befindlichen Zellkultur positioniert ist und den Reaktionsraum von einem Reservoirraum trennt, dessen Aufnahmevolumen um ein Vielfaches größer ist als das Aufnahmevolumen des Reaktionsraums und daß der Trennkörper entweder zwischen der bodennahen, die Zellkultur überdeckenden Meßposition und einer bodenfernen Position, in zumindest der der Reservoirraum mit dem Reaktionsraum in Flüssigkeitsverbindung steht, durch eine im wesentlichen vertikale Bewegung hin-und herbewegbar ist oder daß der Trennkörper durch eine seitliche Bewegung zwischen der Meßposition und einer Position, in welcher der Reaktionsraum in Flüssigkeitsverbindung mit dem Reservoirraum steht, hin-und herbewegbar ist.

In der bodennahen Position bildet der Trennkörper eine Abgrenzung eines kleinvolumigen Reaktionsraums, die zur Diffusionseinschränkung für Substanzen dient, die von den Zellen gebildet oder verbraucht werden.
Mit dieser erfindungsgemäßen Maßnahme ist ein kleinvolumiger Reaktionsraum realisierbar, in dem die durch den Zellstoffwechsel bedingten, meßbaren stofflichen Änderungen viel rascher vonstatten gehen, als in einem großen Volumen. Aus der Änderungsgeschwindigkeit des pH-Wertes oder des Sauerstoff-Partialdruckes in einem Zeitintervall lassen sich beispielsweise Informationen über die Aktivität des Zellstoffwechsels gewinnen.
Zweckmäßigerweise sind dabei ein oder mehrere Sensoren und/oder Meßeinrichtungen im Bereich dieses Reaktionsraums angeordnet.

Eine Ausführungsform der Erfindung sieht vor, daß voneinander getrennte Kulturbereiche insbesondere durch strukturiert aufgebrachte, zellabweisende Beschichtungen oder durch eine dreidimensionale Strukturierung des Bodens mit Vertiefungen beziehungsweise Erhöhungen in den dazwischen befindlichen Trennbereichen gebildet sind und daß in den Kulturbereichen das Kulturmedium vorzugsweise jeweils als Tropfen vorliegt.

Mit dem zum Beispiel stempelartigen, eine unterseitige Flachseite aufweisenden Trennkörper kann der Tropfen von oben beaufschlagt und ein Teilvolumen seitlich verdrängt werden, bis unterhalb des Trennkörpers nur ein dünner, die Zellkultur überdeckender Flüssigkeitsfilm verbleibt, der den Reaktionsraum mit einem Mikroreaktionsvolumen an Kulturmedium bildet.

Der Trennkörper trennt in bodennaher Position den Reaktionsraum von dem übrigen, seitlich nach außen verdrängten Tropfenvolumen des Kulturmediums ab. Durch eine Hubbewegung des Trennkörpers kann dieses seitlich verdrängte, als Reservoir dienende Tropfen volumen mit dem im Reaktionsraum befindlichen Kulturmediumvolumen vermischt und dadurch eine Regeneration des im Reaktionsraum befindlichen Kulturmediums erreicht werden.
Eine Ausgestaltung sieht vor, daß wenigstens ein Trennkörper zu einer oder mehreren, auf dem Boden der Aufnahme befindlichen Zellkultur(en) jeweils seitlich zum Verdrängen eines Teilvolumens des die Zellkultur überdeckenden, flüssigen Kulturmediums positionierbar und dort gegebenenfalls annäherbar ist.
Beispielsweise können auf einem Halbleiterwafer mit Sensoren im Bereich dieser Sensoren Zellkulturen aufgebracht werden, bei denen dann ein Trennkörper nacheinander seitlich so positioniert werden kann, daß er sich oberhalb einer Zellkultur befindet. In dieser Position können dann Messungen durchgeführt werden. Es können dabei auch mehrere, seitlich in etwa horizontaler und gegebenenfalls auch etwa vertikaler Richtung positionierbare Trennkörper vorgesehen sein.
Es können hierbei die Zellkultur(en) auf den Halbleiterwafer aufgebracht werden, um die Sensoren nach der Herstellung des Wafers zu testen oder aber der Halbleiterwafer mit den Sensoren bildet im wesentlichen die Testvorrichtung, um Messungen an Zellkulturen vornehmen zu können.
Der mit Zellkulturen besetzte Bereich des Halbleiterwafers kann bedarfsweise mit einer Umgrenzung zur Bildung eines Behältnisses für Zellkulturmedium umgrenzt sein.

Eine Ausführungsform der Erfindung, für die selbständiger Schutz beansprucht wird, sieht vor, daß als Aufnahme ein oben offenes Behältnis vorgesehen ist, in den ein Trennkörper ragt, der den Gesamtaufnahmeraum des Aufnahmebehältnisses in zwei übereinanderliegende Teilräume aufteilt, daß der bodenseitige Teilraum einen gegenüber dem Gesamtaufnahmevolumen des Aufnahmebehältnisses kleinvolumigen Reaktionsraum und der andere Teilraum einen Reservoirraum bildet, daß wenigstens ein Strömungskanal vorgesehen ist, der einerseits an den Reaktionsraum und andererseits an den Reservoirraum angeschlossen ist, daß innerhalb des Trennkörpers ein oder mehrere Durchtrittskanäle vorgesehen sind, die in den kleinvolumigen Teilraum des Aufnahmebehältnisses und/oder den Reservoirraum des Aufnahmebehältnisses münden.
Zweckmäßigerweise sind auch hierbei ein oder mehrere Sensoren und/oder Meßeinrichtungen im Bereich des Reaktionsraumes angeordnet.
Auch bei dieser Ausführungsform können durch den kleinvolumigen Reaktionsraum zum Beispiel mikrosensorische Messungen bei erhöhter Zellkonzentration durchgeführt werden, wobei die erhöhte Zellkonzentration zu schnelleren Aktivitätsänderungen führt und damit zu einer Beschleunigung der entsprechenden Messung.
Der oberhalb des Reaktionsraums befindliche, durch den Trennkörper getrennte Resevoirraum kann bei dieser Ausführungsform vergleichsweise groß sein, so daß verbrauchtes Medium im Reaktionsraum durch konvektive Vermischung mit Kulturmedium aus dem Reservoirraum über längere Zeiträume regenerierbar ist, ohne daß von außen Kulturmedium zugeführt werden muß.
Das konvektive Vermischen des im Reaktionsraum und im Reservoirraum befindlichen Mediums kann erfolgen, indem über den Durchtrittskanal ein bestimmtes Flüssigkeitsquantum an Kulturmedium dem Reaktionsraum zugeführt und wieder abgesaugt wird. Die konvektive Vermischung erfolgt über den Strömungskanal.
Nach einer Ausführungsform der Erfindung ist vorgesehen, daß der Trennkörper innerhalb des Aufnahmebehältnisses zwischen einer bodennahen Position und einer bodenfernen Position hin-und herbewegbar ist.
Zum Regenerieren der Zellkulturlösung im Reaktionsraum besteht dadurch die Möglichkeit, den stempelförmigen Körper anzuheben und wieder in seine vorherige Position abzusenken. Dabei findet eine konvektive Vermischung des verbrauchten Mediums aus dem Reaktionsraum und dem restlichen, im Reservoirraum befindlichen Medium des Zellkulturbehältnisses über den Strömungskanal statt. Das Abgeben und Ansaugen eines Flüssigkeitsquantums in Bezug auf das Mikroreaktionsvolumen erfolgt somit durch eine mechanische Bewegung des Trennkörpers nach oben und unten.
Ein Komplettaustausch des Zellkulturmediums wird in Abhängigkeit von der Stoffwechselaktivität der Zellen und dem Gesamtvolumen der Meßlösung erst in größeren Zeitabständen fällig.

Der Trennkörper bildet einen oberen Abschluß des Reaktionsraum, so daß ein Schutz vor Verdunstung und auch Kontamination gegeben ist. Im übrigen wird das darin befindliche Mikroreaktionsvolumen auch durch das wesentlich größere Volumen von Kulturmedium im Reservoirraum gepuffert, so daß auftretende Verdunstungen an der Flüssigkeitsoberfläche insgesamt keine Auswirkungen haben auf das Mikroreaktionsvolumen.

Eine vorteilhafte Ausgestaltung der Erfindung sieht vor, daß der Abstand des Trennkörpers von der Zellkultur und damit die bodennahe Position des Trennkörpers innerhalb des Aufnahmebehältnisses einstellbar ist.
Durch den in seinem Abstand zum Boden des Aufnahmebehältnisses einstellbaren Trennkörper kann in der Umgebung der Zellkultur einden jeweiligen Anforderungen exakt angepasstes Mikroreaktionsvolumen eingestellt werden. Dieses Mikroreaktionsvolumen kann dabei so eingestellt werden, daß nur ein schmaler Flüssigkeitssaum von z.B. 50 µm bis 500 µm über der Zellkultur verbleibt. Dadurch wird das geforderte Mikroreaktionsvolumen mit entsprechend hoher Zellkonzentration hergestellt.

Eine vorteilhafte Ausgestaltung der Erfindung sieht vor, daß die Unterseite des Trennkörpers eine Gasblasen nach außen ableitende Profilierung, vorzugsweise eine konvexe Wölbung aufweist. Luft- bzw. Gasblasen können somit entweichen und beeinträchtigen die Zellkultur beziehungsweise die Messung durch die Sensoren nicht. Der Trennkörper bildet dabei keinen luft- und flüssigkeitsdichten Abschluß des Zellkulturbereiches, so daß die Gasblasen nach außen entweichen können. Dadurch ist auch die Montage erleichert, weil dazu nicht auf Gasfreiheit geachtet werden muß.

Besonders vorteilhaft ist es, wenn eine Vielzahl von Aufnahmebehältnissen vorzugsweise als Teil eines Pipettierautomaten vorgesehen sind, daß diese Aufnahmebehältnisse insbesondere durch handelsübliche Multiwell-Platten gebildet sind und daß am unteren Ende der Pipetten oder Dispenser-Kanäle des Pipettierautomaten jeweils die Trennkörper vorgesehen sind. Dadurch bilden diese Trennkörper eine Sonderform einer Pipettenspitze.

Gerade im Zusammenhang mit einem Pipettierautomaten und einer entsprechenden Vielzahl von zum Beispiel 96 Aufnahmebehältnissen als Zellkulturgefäße, wirkt sich der einfache Aufbau der jeweiligen Vorrichtung mit dem stempelförmigen Körper vorteilhaft aus. Insbesondere können zum Beispiel mikrosensorische Messungen in solchen Standard-Zellkulturformationen durchgeführt werden, was bei bekannten Systemen mit pumpengetriebenen Durchfluß praktisch nicht möglich ist.

Nach einer Weiterbildung der Erfindung ist vorgesehen, daß beabstandet zum Behältnis-Boden und zwischen diesem und dem in der bodennahen Position befindlichen, Trennkörper eine flüssigkeitsdurchlässige Membrane oder dergleichen Filter oder Schutzabdeckung für die Zellkultur vorgesehen ist.
Durch die Integration einer solchen Membran, die als mikroporöses Membranfilter ausgebildet sein kann oder einer anderen Schutzeinrichtung unmittelbar oberhalb der Zellkultur, wird die empfindliche Zellkultur vor Flüssigkeits-Scherkräften und Druckstößen, die beim konvektiven Vermischen und dem Zuführen und Absaugen von Zellkulturmedium auftreten können, bewahrt. Auch können dann Messungen an nicht-adhärenten Zelltypen durchgeführt werden, die ansonsten leicht von den Flüssigkeitsströmen weg von den Sensoren oder aus dem kleinvolumigen Teilraum gespült werden könnten.

Nach einer Ausgestaltung der Erfindung kann die Auflagefläche für die Zellkultur optisch transparent sein. Damit können dann Messungen zum Beispiel der Absorption, Streuung, Fluoreszens von den Zellen, gegebenenfalls nach einer Anfärbung, durchgeführt werden.

Zusätzliche Ausgestaltungen der Erfindung sind in den weiteren Unteransprüchen aufgeführt. Nachstehend ist die Erfindung mit ihren wesentlichen Einzelheiten anhand der Zeichnungen noch näher erläutert.

Es zeigt, etwas schematisiert:
- Fig. 1: eine Vorrichtung für Untersuchungen an Zellkulturen miteinem Aufnahmebehältnis, einer darin befindlichen Zellkultur sowie flüssigem Kulturmedium und einem in dieses eintauchenden Trennkörper,
- Fig.2 bis 4: die in Fig. 1 gezeigte Vorrichtung in unterschiedlichen Betriebssituationen,
- Fig.5 bis 7: die in Fig. 1 gezeigte Vorrichtung mit in unterschiedlichen Positionen befindlichen Trennkörper,
- Fig. 8: eine schematische Darstellung eines Pipettierautomaten,
- Fig. 9: eine Vielzahl von Aufnahmebehältnissen in Form einer Multititerplatte mit Mikrosensorarrays,
- Fig.10 und 11: auf einem Aufnahmeboden befindliche Kulturmediumtropfen mit in unterschiedlichen Positionen befindlichen Trennkörpern.

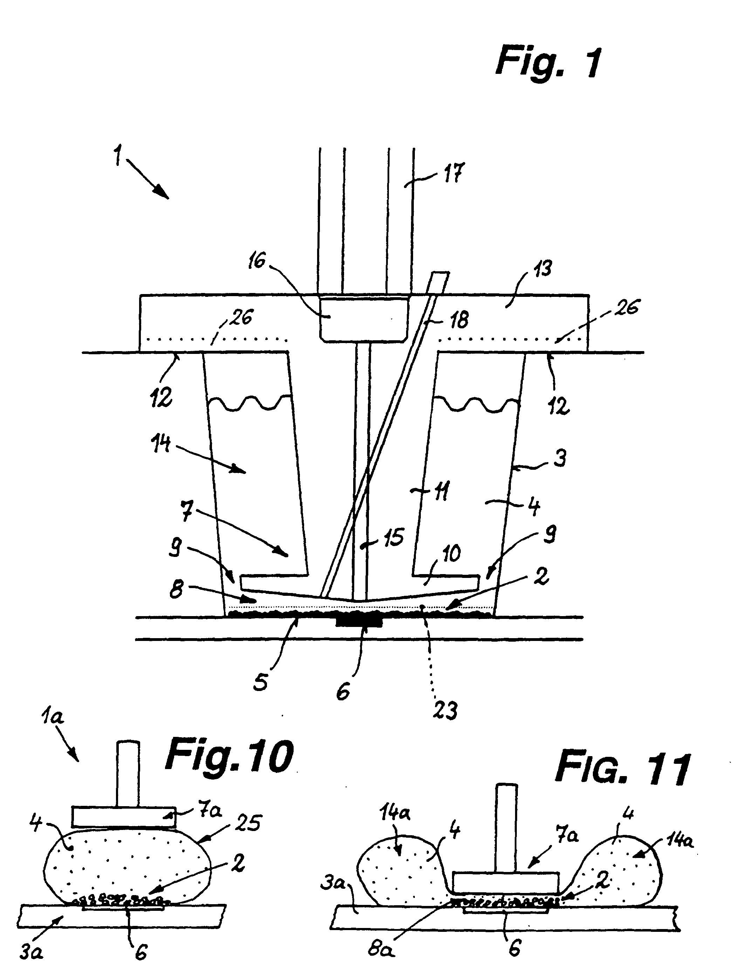

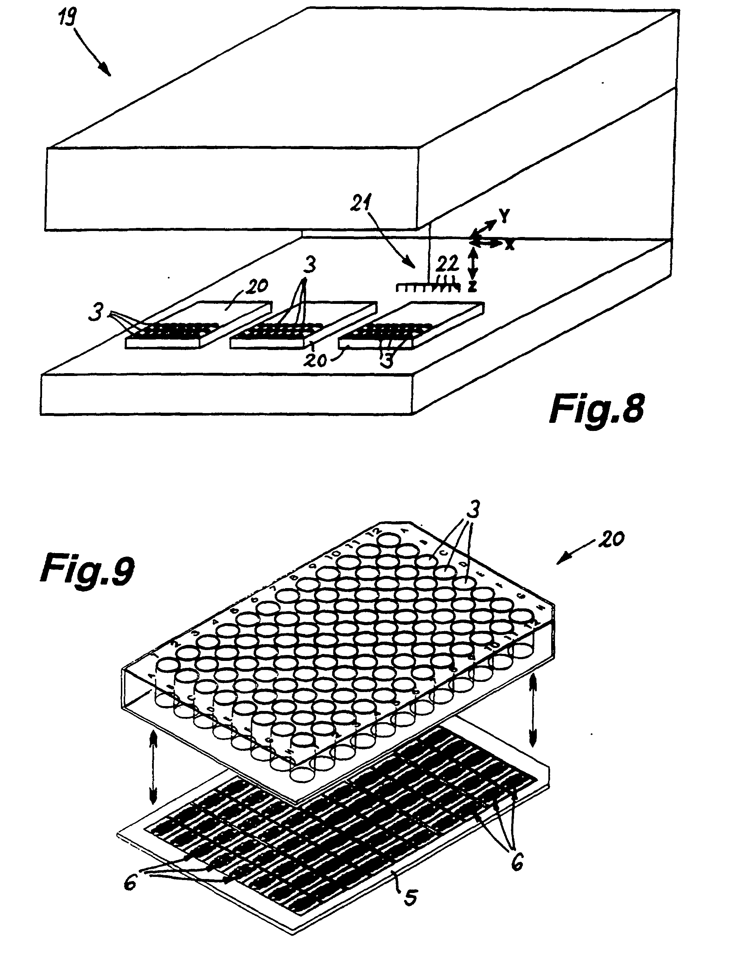

Eine in Fig. 1 bis 7 gezeigte Vorrichtung 1 dient zur Untersuchung an Zellkulturen 2. Die Vorrichtung weist dazu ein vorzugsweise trogförmiges Aufnahmebehältnis 3 auf, in welchem sich bodenseitig die Zellkultur 2 in einem flüssigen Kulturmedium 4 befindet. Am Boden 5 des Aufnahmebehältnisses befinden sich ein oder mehrere Sensoren 6, mit denen Messungen an der Zellkultur 2 vorgenommen werden können. Bevorzugt ist ein Halbleiterchip mit insbesondere einer Vielzahl von Mikrosensoren vorgesehen, wobei auch der gesamte Boden als Sensor-Chip ausgebildet sein kann, wie dies gut in Fig. 9 erkennbar ist. Der Sensor-Chip kann verschiedene Sensor-Funktionen tragen, beispielsweise für die Ionenaktivität, die Sauerstoffaktivität, die Aktivität gelöster Metabolite, elektrische Inpedanz, Temperatur und dergleichen.

Das Aufnahmebehältnis 3 ist oben offen und es ist dort ein Trennkörper 7 eingeführt, der in einer bodennahen Position, wie in Fig. 1 erkennbar, einen gegenüber dem Gesamtaufnahmevolumen des Aufnahmebe hältnisses klein volumigen Teilraumals Reaktionsraum 8 begrenzt. Der Trennkörper 7 hat einen etwa dem Querschnitt des Aufnahmebehältnisses angepassten Querschnitt und zwischen ihm und der Innenwand des Aufnahmebehältnisses ist ein Überströmspalt 9 als Strömungskanal gebildet. Bei einem Behälterdurchmesser von beispielsweise 5 mm oder 10 mm weist der Überströmspalt 9 eine lichte Weite von weniger als 1 mm auf.

Der Trennkörper 7 hat im Ausführungsbeispiel einen plattenförmigen Kopf 10, an den sich ein stielförmiger Schaft 11 anschließt. Die der Zellkultur zugewandte Seite des Trennkörpers beziehungsweise dessen Kopf 10 ist so dimensioniert, daß sie zumindest eine den für die Messung der durch die Zellen verbrauchten oder gebildeten Substanzen Fläche abdeckt beziehungsweise begrenzt und insbesondere eine etwa der Sensor-Oberfläche entsprechende Fläche aufweist.
Außerhalb des Aufnahmebehältnisses 3 ist der Schaft 11 mit einem den Behältnisrand 12 übergreifenden Deckel 13 verbunden, der insbesondere einen Anschlag bildet zur Begrenzung der Eintauchtiefe des Trennkörpers 7 ins Innere des Aufnahmebehältnisses 3. Die Auflageseite des Deckels 3 ist so ausgebildet, daß sich kein gasdichter Abschluß ergibt. Strichpunktiert sind entsprechende Profilierungen oder Kanäle 26 angedeutet. Am Behältnisrand 12 und/oder im Auflagebereich des Deckels 13 können Einstellmittel vorgesehen sein, durch die die bodennahe Position des Trennkörpers 7 innerhalb des Aufnahmebehältnisses einstellbar ist. Dementsprechend ist die Größe des Reaktionsraums variierbar. Insbesondere kann dadurch ein sehr kleines Mikroreaktionsvolumen eingestellt werden, wobei der Abstand der Unterseite des Kopfes 10 von der Zellkultur zum Beispiel 50 µm bis 500 µm betragen kann.
Der oberhalb des Kopfes 10 befindliche Teilraum des Aufnahmebehältnisses 3 bildet einen Reservoirraum 14, in welchem sich ebenso wie in dem unteren, kleinvolumigen Reaktionsraum 8 flüssiges Kulturmedium 4 befindet. Das Aufnahmevolumen des Reservoirraumes kann etwa 100 mal größer sein als das Aufnahmevolumen des Reaktionsraums 8.

Innerhalb des Trennkörpers 7 ist ein in den Reaktionsraum 8 mündender Durchtrittskanal 15 vorgesehen, dessen anderes Ende im Bereich des Deckels 13 einen Anschluß 16 zum Verbinden mit einer Pipette 17 oder dergleichen aufweist. Über diesen Durchtrittskanal 15 kann frisches Kulturmedium zugeführt und verbrauchtes Medium abgesaugt werden.
Zusätzlich zu dem in den Reaktionsraum 8 mündenden Durchtrittskanal 15 können auch in den Reservoirraum 14 mündende Durchtrittsoder Verbindungskanäle vorgesehen sein.
Mit 18 ist eine Elektrode oder ein Sensor bezeichnet, die beziehungsweise der durch den Trennkörper 7 hindurchgeführt ist und bei dem Teilraum 8 endet. Beispielsweise kann hier eine Bezugselektrode für eine pH- und pO₂-Messung eingesetzt werden.

Die erfindungsgmäße Vorrichtung 1 wird bevorzugt in Verbindung mit einem Pipettierautomaten 19 eingesetzt, der schematisch in Fig. 8 gezeigt ist. Deutlich sind hier drei sogenannte Multiwell-Platten 20 mit einer Vielzahl von Aufnahmebehältnissen 3 erkennbar. Standardmäßig können solche Multiwell-Platten 6, 24, insbesondere aber auch 96 Aufnahmebehältnisse als Zellkulturgefäße aufweisen.

Ein Pipettenkopf 21 mit einer Anzahl von Dispenser-Kanälen 22 ist mit einer hier nicht näher dargestellten Positioniereinrichtung verbunden, mittels der der Pipettenkopf in einer Ebene in zwei Koordinatenrichtungen bewegbar und zusätzlich auch in der Höhe verstellbar ist. Die einzelnen Dispenser-Kanäle 22 sind mit ihren freien Enden jeweils mit einem Trennkörper 7 verbunden und so positionierbar, daß die Trennkörper 7 in einer oder mehreren Reihen gleichzeitig in die Aufnahmebehältnisse 3 der Multiwell-Platten 20 eingeführt werden können.

Mit Hilfe des Pipettierautomaten kann in vorgebbaren Zeitabständen Flüssigkeit aus den einzelnen Aufnahmebehältnissen 3 entnommen und in andere Gefäße überführt werden beziehungsweise es kann Flüssigkeit aus Gefäßen entnommen und den mit Zellkulturen versehenen Aufnahmebehältnissen zugegeben werden. Prinzipiell kann somit eine periodische Regeneration des Mikroreaktionsvolumens in dem kleinvolumigen Teilraum 8 und somit in der Umgebung der Sensoren 6 sowie der lebenden Zellkultur vorgenommen werden. Das Mikroreaktionsvolumen wird hierbei nicht durch eine geschlossene Perfusionskammer und die Regeneration nicht durch das Hindurchfließenlassen einer Lösung wie beim Stand der Technik erzeugt, sondern durch die Ausbildung des Trennkörpers 7 in Verbindung mit den nachfolgenden Verfahrensschritten erreicht.

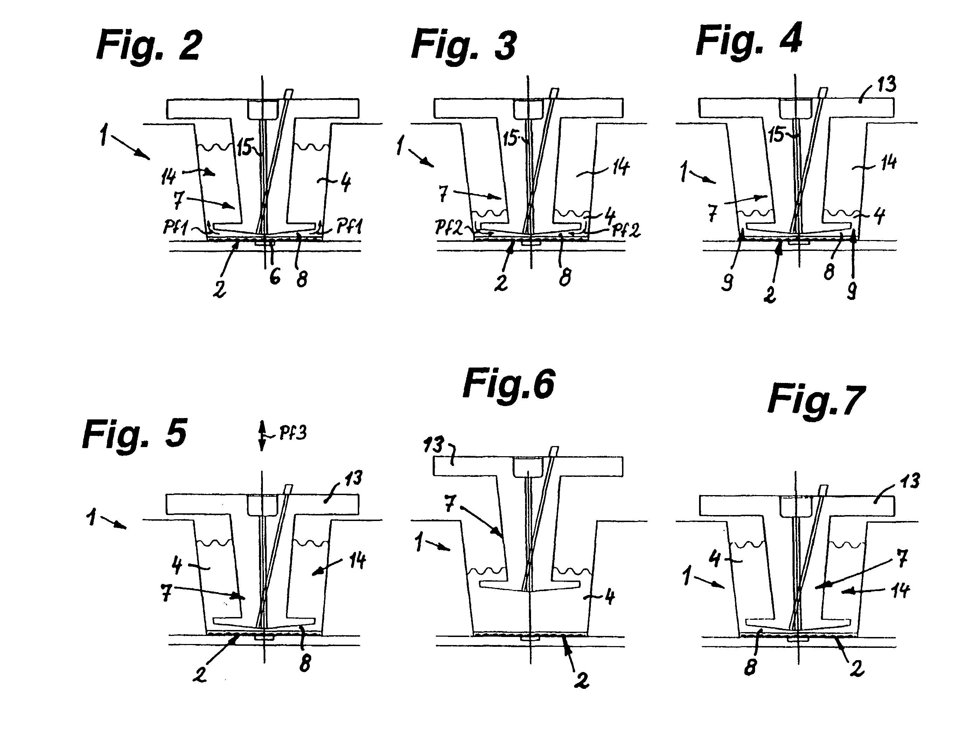

Dazu ist in den Fig. 2 bis 4 ein Betriebsmodus gezeigt, bei dem ein Durchmischen des flüssigen Zellkulturmediums durch periodische Abgabe und Aufnahme von Zellkulturmedium erfolgt.

Der andere Betriebsmodus ist anhand der Fig. 5 bis 7 wiedergegeben. Hier erfolgt das Durchmischen des Zellkulturmediums durch periodisches Absenken und Anheben des Trennkörpers 7.

In den Fig. 2 bis 7 ist der jeweils mit dem Anschluß 16 verbundene Dispenser-Kanal 22 der Einfachheit halber weggelassen. Über diesen erfolgt das Zugeben und Absaugen von Kulturmedium. Eine Pipette 17 beziehungsweise Dispenser-Kanäle 22 sind in den Fig. 1 beziehungsweise 8 erkennbar.

Bei dem Betriebsmodus gemäß Fig. 2 bis 4 wird der am unteren Ende eines Dispenser-Kanales 22 eines Pipettenkopfes 21 befindliche Trennkörper 7 in ein Aufnahmebehältnis 3 eingeführt und der Zellkultur 2 soweit angenähert, daß sein Kopf 10 oberseitig einen kleinvolumigen Reaktionsraum 8 begrenzt, wobei das Flüssigkeitsvolumen beispielsweise wenige Mikroliter betragen kann. In dieser bodennahen Position des Trennkörpers 7 beziehungsweise seines Kopfes 10 kann dann zum Beispiel eine mikrosensorische Messung beispielsweise der Stoffwechselaktivität der Zellkultur 2 erfolgen. Dabei können aus der Änderungsgeschwindigkeit des pH-Wertes oder des Sauerstoff-Partialdruckes in diesem Meßintervall Informationen über die Aktivität des Zellstoffwechsels gewonnen werden.

In einem nächsten Zeitintervall erfolgt dann eine Regeneration des Kulturmediums innerhalb des im Reaktionsraum 8 befindlichen Mikroreaktionsvolumens. Über den Durchtrittskanal 15 wird dazu ein bestimmtes Flüssigkeitsquantum an Kulturmedium dem Reaktionsraum 8 zugeführt, wobei Kulturmedium von dem Reaktionsraum 8 gemäß den Pfeilen Pf1 in den Reservoirraum 14 verdrängt wird, wo eine konvektive Vermischung von frischem und verbrauchtem Medium stattfindet. Anschließend wird ein gleiches Flüssigkeitsquantum durch den Kanal 15 abgesaugt, so daß aus dem Reservoirraum 14 gemäß den Pfeilen Pf2 Kulturmedium aus dem Reservoirraum 14 in den Reaktionsraum 8 überströmt. Anschließend erfolgt dann in einem Ruheintervall (Fig. 4), wo keine Durchströmung vorhanden ist, eine nächste zum Beispiel mikrosensorische Messung. In diesem anhand der Fig. 2 bis 4 beschriebenen Betriebsmodus wird im wesentlichen nur das Mikroreaktionsvolumen periodisch ausgetauscht, nicht jedoch das Reservoirvolumen des Aufnahmebehältnisses 3. Durch das Zuführen und Absaugen von dem Flüssigkeitsquantum wird praktisch eine "Pumpwirkung" mit Durchmischen des in dem Reaktionsraum 8 und in dem Reservoirraum 14 befindlichen, flüssigen Zellkulturmediums erreicht.
In vorgebbaren Zeitabständen wird ein Flüssigkeitsquantum entnommen und dieses in ein Abfallgefäß überführt und anschließend wird frisches Medium aus einem Vorratsgefäß angesaugt und in das mit einer Zellkultur bestückte Aufnahmebehältnis 3 gefüllt.

Der Betriebsmodus gemäß Fig. 5 bis 7 sieht vor, daß der Trennkörper 7 durch eine vertikale Bewegung gemäß dem Doppelpfeil Pf3 in periodischen Zeitabständen nach oben und unten geführt wird. Befindet sich der Trennkörper 7 in der unteren Position (Fig. 5 und 7) ist das vorgesehene Mikroreaktionsvolumen innerhalb des Reaktionsraums 8 für die Messung vorhanden. Nach dem Meßintervall wird der Trennkörper 7 angehoben und durch diese Bewegung nach oben findet eine konvektive Vermischung des ursprünglich im Reaktionsraum 8 befindlichen, verbrauchten Kulturmediums mit dem restlichen Kulturmedium des Reservoirraumes 14 und damit eine Homogenisierung des Zellkulturmediums statt. Dieser Prozeß wiederholt sich periodisch. Ein Komplettaustausch des Zellkulturmediums wird nur in jeder n-ten Periode fällig, abhängig von der Stoffwechselaktivität der Zellen und dem Volumen der Meßlösung.
Das Regenerieren des im Reaktionsraum 8 befindlichen Kulturmediums erfolgt in diesem Fall durch eine mechanische Bewegung des stempelförmigen Körpers und durch Vermischen mit unverbrauchtem Kulturmedium aus dem Reservoirraum 14.

In beiden Betriebsmodi steht durch das im Vergleich zum Reaktionsraum 8 große Reservoir-Volumen insgesamt eine vergleichsweise große Menge an Zellkulturmedium zur Verfügung, das durch konvektionelle Vermischung mit dem Zellkulturmedium aus dem Mikroreaktionsvolumen für eine vergleichsweise lange mögliche Verweildauer sorgt, bis schließlich ein Austausch des Kulturmediums erfolgen muß.
Durch die erfindungsgemäße Anordnung, die auf ein kontinuierliches oder intervallweises Hindurchfließenlassen von Zellkulturlösungen verzichtet, werden die damit verbundenen Probleme vermieden. Die Anordnung kann mit einfachen Mitteln so gestaltet werden, daß sie kompatibel zu weitverbreiteten Zellkulturformaten wie z.B. die 96 well-Multititerplatte ist sowie zu einer Installation in Zellkultur-Inkubatoren ist. Sie kann sinnvollerweise, wie bereits vorbeschrieben, mit einem Pipettierautomaten kombiniert werden, um eine nicht-manuelle Regeneration der Zellkulturlösungen zu erreichen.

In den Fig. 1 bis 7 ist noch gut erkennbar, daß die Unterseite des Kopfes 10 des Trennkörpers 7 konvex gewölbt ist, um zu bewirken, daß Gasblasen seitlich nach außen abgeleitet und über den Überströmspalt 9 nach oben gelangen können. Da der Deckel 13 keinen dichtenden Abschluß des Aufnahmebehältnisses 3 bildet, was durch die punktierten Kanäle 26 in Fig. 1 angedeutet ist, ist ein Gasaustausch zwischen Kulturmedium und der umgebenden Atmosphäre gewährleistet. Trotz dieses nicht gasdichten Abschlusses des Aufnahmebehältnisses 3 ist ein genügender Schutz vor Verdunstung der Zellkulturlösung vorhanden und es wird auch das Eindringen mikrobiologischer Kontaminationen verhindert. Die Kanäle 26 sind in ihrem Querschnitt entsprechend klein dimensioniert.
Der gesamte Trennkörper 7 mit Deckel 15 besteht vorzugsweise aus glattem, zellabweisenden, inerten und leicht sterilisierbarem Material.
Weiterhin ist in den Figuren 1 bis 7 erkennbar, daß beabstandet zum Behältnis-Boden und zwischen diesem und dem in der bodennaher Position befindlichen Trennkörper 7 eine mikroporöse Membrane 23 als Schutzabdeckung für die Zellkultur vorgesehen ist.
Durch dieses mikroporöse Membranfilter wird die empfindliche Zellkultur vor Flüssigkeits-Scherkräften und Druckstößen, die beim konvektiven Vermischen und dem Zuführen und Absaugen von Zellkulturmedium auftreten können, geschützt. Insbesondere bei nicht-adhärenten Zelltypen wird vermieden, daß diese nicht von den Flüssigkeitsströmen weggespült werden.

Fig. 9 zeigt eine Multiwellplatte 20 vor dem Zusammenfügen von einem Oberteil und einem Unterteil. In dem hier dargestellten Ausführungsbeispiel wird für die Aufnahmebehältnisse 3 von einer handelsüblichen Mikrotiterplatte der Bodenbereich abgetrennt, so daß sich durchgängige Röhrchen ergeben. Dieses Mikrotiterplatten-Oberteil wird dann auf eine Substratplatte 24 aufgesetzt und vorzugsweise durch Ultraschallschweißen dicht mit dieser verbunden. Die Substratplatte 24 mit den jeweiligen Sensoren bildet dann den Boden der Einzelbehältnisse 3. Die Sensoren oder Sensor-Arrays auf der Substratplatte 2 sind bei Verwendung einer Mikrotiterplatte in dem Abstand der einzelnen Röhrchen oder Einzelbehältnisse angeordnet.

Durch Verwendung eines Mikrotiterplatten-Oberteiles zur Bildung der Aufnahmebehältnisse 3 besteht die Möglichkeit, bisher im Zusammenhang mit handelsüblichen Mikrotiterplatten eingesetzte Apparaturen, beispielsweise den in Fig.8 gezeigten Pipettierautomaten beziehungsweise einen Beprobungsautomaten, einen Mikroplattenreader und dergleichen unverändert einsetzen zu können.
Die Sensoren 6 können durch Sensorarrays mit mehreren, unterschiedlichen Einzelsensoren gebildet sein. Auf der Substratplatte kann sich auch eine Steuer- und Auswerteeinrichtung oder Teile davon befinden.
Außer elektronischen Sensoren auf Halbleiterbasis können auch noch andere Sensoren, beispielsweise auf optischer Basis oder auf der Basis der Dickfilmtechnologie oder biologische Sensoren vorgesehen sein und vorzugsweise in Kombination mit den zuvor beschriebenen Sensoren eingesetzt werden.
Weiterhin besteht die Möglichkeit, unterseitig bei der Halbleiter-Substratplatte 24 eine Heizschicht vorgesehen, mittels der eine Temperierung der Substratplatte möglich ist. Bei Zellkulturen können so auch bezüglich der Temperatur deren normale Lebensbedingungen geschaffen werden, so daß Untersuchungen über einen längeren Zeitraum möglich sind. Es besteht auch die Möglichkeit, anstatt einer durchgängigen Heizschicht partielle, voneinander getrennte Abschnitte von Heizschichten vorzusehen, um bedarfsweise unterschiedliche Temperaturen in bestimmten Bereichen erzeugen zu können. Zur thermostatischen Regelung der Heizung können an einer oder mehreren Stellen der Substratplatte Temperaturmeßsensoren vorgesehen sein. Solche Temperaturmeßsensoren können auch direkt bei den den einzelnen Aufnahmebehältnissen zugeordneten Sensoren 6 integriert sein. Temperaturmeßsensoren im Bereich der Einzelbehältnisse können außer zur thermostatischen Regelung einer Heizung auch zum Erfassen der biologischen Aktivität der Zellen verwendet werden. Die Fig. 10 und 11 zeigen eine Ausführungsform der erfindungsgemäßen Vorrichtung 1a, bei der eine plattenförmige Aufnahme 3a vorgesehen ist, die oberseitig die Sensoren 6 trägt. Im Bereich des oder der Sensoren 6 ist das flüssige Kulturmedium 4 als Tropfen 25 aufgebracht. Dieser Kulturmedium-Tropfen überdeckt auf den Sensoren 6 befindliche Zellen bzw. eine Zellkultur 2. Zur Bildung eines kleinvolumigen Reaktionsraumes 8a wird der in Fig. 10 oberseitig des Tropfens 15 angesetzte Trennkörper 7a in eine bodennahe Position gebracht, wie dies in Fig. 11 gezeigt ist. Beim Absenken des stempelförmigen Trennkörpers 7a wird der Tropfen 25 verformt und ein Teil seines Volumens seitlich nach außen verdrängt. Unterhalb des Trennkörpers 7a in dessen Projektionsverlängerung verbleibt dann zwischen der Zellkultur 2 und der im wesentlichen flachen Unterseite des Trennkörpers 7a eine dünne Flüssigkeitsschicht mit einem entsprechend geringen Volumen, das wesentlich geringer sein kann, als das seitlich verdrängte Volumen des Tropfens 25.
Es steht somit einerseits, wie anhand von den Ausführungsbeispielen gemäß Fig. 1 bis 7 beschrieben, ein kleinvolumiger Reaktionsraum 8a und durch das seitlich über die Trennkörperprojektion verdrängte Kulturmedium-Volumen ein Reservoir 14a zur Verfügung. Ist das Kulturmedium 4 in dem spaltförmigen Reaktionsraum 8a durch Reaktion der Zellen verbraucht, kann der Trennkörper 7a etwas angehoben werden, wodurch aus den seitlich verdrängten Tropfenbereichen Kulturmedium unterhalb des Trennkörpers 7a gelangt und sich mit dem verbrauchten Kulturmedium vermischt. Anschließend wird der Trennkörper 7a wieder abgesenkt, so daß wieder ein kleinvolumiger Reaktionsraum 8a gebildet ist, wobei sich dann in diesem Bereich regeneriertes Kulturmedium befindet. Es schließt sich dann wieder eine Meßphase an. Diese Anordnung kann vorzugsweise eingesetzt werden, um auf unzersägten (Chip)-Wafern Funktionstests an Sensoren durchzuführen.
Die plattenförmige Aufnahme 3a kann etwa wie die Substratplatte 24 gemäß Fig. 9 ausgebildet sein, wobei die einzelnen Sensorbereiche durch strukturiert aufgebrachte, zellabweisende Beschichtungen voneinander getrennt sein können. In diesem Fall bildet der Boden der Aufnahme einen Teil eines die Sensoren aufweisenden Wafers.

Erwähnt sei noch, daß die plattenförmige Aufnahme 3a (Fig. 10 und 11) bzw. der Boden 5 der Aufnahmebehältnisse 3 (vgl. Fig. 1 bis 7) optisch transparent sein kann, damit die Zellkultur bzw. das in diesem Bereich befindliche Kulturmedium für optische Meßeinrichtungen vorzugsweise von unten zugänglich ist.

## Patentansprüche

1. Vorrichtung zur Durchführung von Untersuchungen an lebenden Zellen, Zellkulturen und dergleichen, insbesondere zur Detektion der Stoffwechselaktivität der Zellen, die sich in einem flüssigen Medium befinden, wobei die Vorrichtung wenigstens eine Aufnahme für flüssiges Medium und die Zellkultur aufweist und wobei ein oder mehrere Meßeinrichtungen und/oder Sensoren für Messungen an der Zellkultur vorgesehen sind und wobei ein bewegbarer Trennkörper vorgesehen ist, der einen Reaktionsraum begrenzt, **dadurch gekennzeichnet, daß** der Trennkörper (7,7a) in Meßposition nahe oberhalb einer auf einem Boden der Aufnahme befindlichen Zellkultur positioniert ist und den Reaktionsraum (8,8a) von einem Reservoirraum trennt, dessen Aufnahmevolumen um ein Vielfaches größer ist als das Aufnahmevolumen des Reaktionsraums (8,8a), und daß der Trennkörper (7,7a) entweder zwischen der bodennahen, die Zellkultur überdeckenden Meßposition und einer bodenfernen Position, in zumindest der der Reservoirraum (14,14a) mit dem Reaktionsraum (8,8a) in Flüssigkeitsverbindung steht, durch eine im wesentlichen vertikale Bewegung hin-und herbewegbar ist oder daß der Trennkörper durch eine seitliche Bewegung zwischen der Meßposition und einer Position, in welcher der Reaktionsraum (8,8a) in Flüssigkeitsverbindung mit dem Reservoirraum (14,14a) steht, hin-und herbewegbar ist.

2. Vorrichtung nach Anspruch 1, **dadurch gekennzeichnet, daß** wenigstens ein Trennkörper zu einer oder mehreren, auf dem Boden der Aufnahme befindlichen Zellkultur(en) (2) jeweils seitlich zum Verdrängen eines Teilvolumens des die Zellkultur überdeckenden, flüssigen Kulturmediums (4) positionierbar und dort gegebenenfalls annäherbar ist.

3. Vorrichtung nach Anspruch 1 oder 2, **dadurch gekennzeichnet, daß** der oder die Sensoren (6) am oder im Boden der Aufnahme (3a) angeordnet sind, daß voneinander getrennte Kulturbereiche durch strukturiert aufgebrachte, zellabweisende Beschichtungen oder durch eine dreidimensionale Strukturierung des Bodens mit Vertiefungen beziehungsweise Erhöhungen in den dazwischen befindlichen Trennbereichen gebildet sind und daß in den Kulturbereichen das Kulturmedium (4) jeweils als Tropfen (25) vorliegt.

4. Vorrichtung nach einem der Ansprüche 1 bis 3, **dadurch gekennzeichnet, daß** der Boden der Aufnahme durch wenigstens einen Teil eines zumindest die Sensoren (6) aufweisenden Wafers gebildet ist.

5. Vorrichtung nach Oberbegriff des Anspruches 1, **dadurch gekennzeichnet, daß** als Aufnahme ein oben offenes Behältnis (3) vorgesehen ist, in den ein Trennkörper (7) ragt, der den Gesamtaufnahmeraum des Aufnahmebehältnisses (3) in zwei übereinanderliegende Teilräume aufteilt, daß der bodenseitige Teilraum einen gegenüber dem Gesamtaufnahmevolumen des Aufnahmebehältnisses (3) kleinvolumigen Reaktionsraum (8) und der andere Teilraum einen Reservoirraum (14) bildet, dessen Aufnahmevorlumen um ein Vielfaches größer ist als das des Reaktionsraumes (8), daß wenigstens ein Strömungskanal (9) vorgesehen ist, der einerseits an den Reaktionsraum (8) und andererseits an den Reservoirraum (14) angeschlossen ist, daß innerhalb des Trennkörpers (7) ein oder mehrere Durchtrittskanäle (15) vorgesehen sind, die in den kleinvolumigen Reaktionsraum (8) des Aufnahmebehältnisses (3) und/oder den Reservoirraum (14) des Aufnahmebehältnisses münden.

6. Vorrichtung nach einem der Ansprüche 1 bis 5, **dadurch gekennzeichnet, daß** ein oder mehrere Sensoren (6) und/oder Meßeinrichtungen im Bereich des Reaktionsraumes (8,8a) angeordnet sind.

7. Vorrichtung nach Anspruch 5 oder 6, **dadurch gekennzeichnet, daß** der Trennkörper (7) innerhalb des Aufnahmebehältnisses (3) zwischen einer bodennahen Position und einer bodenfernen Position hin-und herbewegbar ist.

8. Vorrichtung nach einem der Ansprüche 1 bis 7, **dadurch gekennzeichnet, dass** die dem Boden (5) zugewandte Seite des Trennkörpers (7) zur Messung der Fläche, die durch die von den Zellen verbrauchten oder gebildeten Substanzen abgedeckt oder begrenzt ist, im Wesentlichen der Sensor-Oberfläche entspricht.

9. Vorrichtung nach einem der Ansprüche 5 bis 9, **dadurch gekennzeichnet, daß** der Trennkörper (7) von oben in das Aufnahmebehältnis (3) einführbar ist.

10. Vorrichtung nach einem der Ansprüche 1 bis 9, **dadurch gekennzeichnet, daß** der Abstand des Trennkörpers (7) von der Zellkultur (2) und damit die bodennahe Position des Trennkörpers (7) einstellbar ist.

11. Vorrichtung nach einem der Ansprüche 5 bis 10, **dadurch gekennzeichnet, daß** der Trennkörper (7) stempelförmig ausgebildet ist und einen Kopf (10) mit etwa dem Querschnitt des Aufnahmebehältnisses (3) angepaßten Querschnitt hat, der das Aufnahmebehältnis (3) in den Reaktionsraum (8) und den Reservoirraum (14) aufteilt und daß sich an den Trennkörper-Kopf ein nach außen führender Schaft (11) anschließt, dessen Außenquerschnitt kleiner ist als der lichte Innenquerschnitt des Aufnahmebehältnisses und daß der Zwischenraum zwischen dem Schaft und der Innenwand des Aufnahmebehältnisses (3) den Reservoirraum (14) bildet.

12. Vorrichtung nach einem der Ansprüche 5 bis 11 **dadurch gekennzeichnet, daß** innerhalb des Trennkörpers (7) ein oder mehrere einerseits in den Reaktionsraum (8) und andererseits in den Reservoirraum (14) mündende Strömungskanäle vorgesehen sind.

13. Vorrichtung nach einem der Ansprüche 5 bis 12, **dadurch gekennzeichnet, daß** der Strömungskanal durch einen zwischen dem Trennkörper (7) und der Innenwand des Aufnahmebehältnisses (3) vorgesehen Ringspalt (9) oder eine Randprofilierung gebildet ist und daß dieser Strömungskanal beziehungsweise Strömungskanäle bei einem höhenverstellbaren Trennkörper (7) zumindest im Hubbereich zwischen der bodennahen Position und der bodenfernen Position des Trennkörpers (7) vorhanden ist/sind.

14. Vorrichtung nach einem der Ansprüche 1 bis 13, **dadurch gekennzeichnet, daß** die Unterseite des Trennkörpers (7) eine Gasblasen nach außen ableitende Profilierung, vorzugsweise eine konvexe Wölbung aufweist.

15. Vorrichtung nach einem der Ansprüche 5 bis 14, **dadurch gekennzeichnet, daß** eine Hubbegrenzung für den Trennkörper (7) vorgesehen ist und daß dazu ein in der bodennaher Position wirksamer, vorzugsweise am oberen Behälterrand anliegender Anschlag am Trennkörper (7) vorgesehen ist.

16. Vorrichtung nach Anspruch 15, **dadurch gekennzeichnet, daß** der am oberen Behälterrand anliegende Anschlag am Trennkörper (7) durch einen den Behältnisrand übergreifenden Deckel (13) oder durch einen mit einem Konusabschnitt in einen Gegenkonus der Behälteröffnung eingreifenden Deckel gebildet ist.

17. Vorrichtung nach einem der Ansprüche 1 bis 16, **dadurch gekennzeichnet, daß** der Trennkörper (7) oberseitig eine genormte Aufnahmeöffnung, vorzugsweise einen Aufnahmekonus zum Kuppeln mit einer Pipette, einer Pipettenspitze oder einen Dispenser-Kanal aufweist.

18. Vorrichtung nach einem der Ansprüche 5 bis 17, **dadurch gekennzeichnet, daß** das um ein Vielfaches größere Aufnahmevolumen des Reservoirraums (14,14a) gegenüber dem Aufnahmevolumen des Reaktionsraums (8,8a), sich vorzugsweise wie 10:1 bis etwa 100:1 verhält.

19. Vorrichtung nach einem der Ansprüche 1 bis 18, **dadurch gekennzeichnet, daß** zumindest am Boden des Behältnisses (3) oder der Aufnahme wenigstens ein Chip mit einem oder mehreren Mikrosensoren angeordnet ist.

20. Vorrichtung nach einem der Ansprüche 1 bis 19, **dadurch gekennzeichnet, daß** am Trennkörper (7), dem Reaktionsraum (8) und/oder dem Reservoirraum (14) zugewandt, Sensoren (6) und/oder Elektroden vorgesehen sind.

21. Vorrichtung nach einem der Ansprüche 5 bis 20, **dadurch gekennzeichnet, daß** beabstandet zum Behältnis-Boden und zwischen diesem und dem in der bodennaher Position befindlichen Trennkörper (7) eine mikroporöse Membrane (23) oder dergleichen Filter oder Schutzabdeckung für die Zellkultur (2) vorgesehen ist.

22. Vorrichtung nach einem der Ansprüche 1 bis 21, **dadurch gekennzeichnet, daß** der Trennkörper (7) aus glattem, zellabweisenden, inerten und leicht sterilisierbarem Material, vorzugsweise aus Polytetrafluoräthylen besteht.

23. Vorrichtung nach einem der Ansprüche 1 bis 22, **dadurch gekennzeichnet, daß** die Auflagefläche für die Zellkultur (2) optisch transparent ist und daß der Auflagefläche eine optische Meßeinrichtung zugeordnet ist, die vorzugsweise unterseitig angeordnet ist.

24. Vorrichtung nach einem der Ansprüche 5 bis 23, **dadurch gekennzeichnet, daß** eine Vielzahl von Aufnahmebehältnissen (3), vorzugsweise als Teil eines Pipettierautomaten (19) vorgesehen sind, daß diese Aufnahmebehältnisse (3) durch handelsübliche Multiwell-Platten (20) gebildet sind und daß an den unteren Enden der Dispenser-Kanäle des Pipettierautomaten jeweils die Trennkörper (7) vorgesehen sind.

25. Vorrichtung nach einem der Ansprüche 1 bis 24, **dadurch gekennzeichnet, daß** der Trennkörper (7) an seiner dem Boden abgewandten Seite einen Anschluß zum Verbinden, insbesondere zum Aufstecken auf einen Dispenserkanal (22) vorzugsweise eines Pipettierautomaten aufweist.

## Claims

1. An apparatus for performing analyses on living cells, cell cultures and the like, in particular for the detection of the metabolic activity of the cells which are located in a fluid medium, the apparatus comprising at least one receiver for fluid medium and the cell culture and one or more measuring instruments and/or sensors being provided for measurements on the cell culture and a movable separating member being provided, which delimits a reaction chamber,
**characterised in that** the separating member (7, 7a) is positioned in the measuring position close above a cell culture located on a bottom of the receiver and separates the reaction chamber (8, 8a) from a reservoir chamber, the volume of which is greater by a multiple than the volume of the reaction chamber (8, 8a),
and **in that** the separating member (7, 7a) can be moved to and fro by a substantially vertical movement either between the measuring position close to the bottom and covering the cell culture and a position far from the bottom, in at least which the reservoir chamber (14, 14a) is in fluid communication with the reaction chamber (8, 8a)
or **in that** the separating member can be moved to and fro by a lateral movement between the measuring position and a position in which the reaction chamber (8, 8a) is in fluid communication with the reservoir chamber (14, 14a).

2. An apparatus according to Claim 1,
**characterised in that** at least one separating member for one or more cell culture(s) situated on the bottom of the receiver can be positioned in each case laterally to displace a partial volume of the liquid culture medium (4) covering the cell culture and if necessary can be brought closer there.

3. An apparatus according to Claim 1 or 2,
**characterised in that** the sensor or sensors (6) are disposed at or in the bottom of the receiver (3a),
**in that** culture regions separated from one another are formed by cell-repellent coatings applied in structured manner or by a three-dimensional structuring of the bottom with recesses or raised portions in the separating regions located between them
and **in that** the culture medium (4) is present as a drop (25) in the culture regions.

4. An apparatus according to one of Claims 1 to 3,
**characterised in that** the bottom of the receiver is formed by at least one part of a wafer comprising at least the sensors (6).

5. An apparatus according to the pre**characterising** clause of Claim 1,
**characterised in that** a receptacle (3) open at the top is provided as the receiver, into which a separating member (7) protrudes, which divides the entire volume of the receptacle (3) into two overlapping sub-chambers,
**in that** the sub-chamber close to the bottom forms a reaction chamber (8) having a small volume in comparison with the overall volume of the receptacle (3) and the other sub-chamber forms a reservoir chamber (14), the volume of which is greater by a multiple than that of the reaction chamber (8),
**in that** at least one flow duct (9) is provided, which is firstly connected to the reaction chamber (8) and secondly to the reservoir chamber (14),
**in that** inside the separating member (7) one or more through-ducts (15) are provided, which discharge into the small-volume reaction chamber (8) of the receptacle (3) and/or the reservoir chamber (14) of the receptacle.

6. An apparatus according to one of Claims 1 to 5,
**characterised in that** one or more sensors (6) and/or measuring instruments are disposed in the region of the reaction chamber (8, 8a).

7. An apparatus according to Claim 5 or 6,
**characterised in that** the separating member (7) can move to and fro inside the receptacle (3) between a position close to the bottom and a position far from the bottom.

8. An apparatus according to one of Claims 1 to 7,
**characterised in that** for the measurement of the area which is covered or delimited by the substances used or formed by the cells, the side of the separating member (7) facing the bottom (5) substantially corresponds to the sensor surface.

9. An apparatus according to one of Claims 5 to 9,
**characterised in that** the separating member (7) can be introduced from above into the receptacle (3).

10. An apparatus according to one of Claims 1 to 9,
**characterised in that** the distance of the separating member (7) from the cell culture (2) and thus the position of the separating member (7) close to the bottom is adjustable.

11. An apparatus according to one of Claims 5 to 10,
**characterised in that** the separating member (7) has a plunger-shaped construction and has a head (10) having a cross section adapted roughly to the cross section of the receptacle (3), which divides the receptacle (3) into the reaction chamber (8) and the reservoir chamber (14)
and **in that** connected to the separating member head is an outwardly leading shaft (11), the external cross section of which is smaller than the clear internal cross section of the receptacle
and **in that** the space between the shaft and the inner wall of the receptacle (3) forms the reservoir chamber (14).

12. An apparatus according to one of Claims 5 to 11,
**characterised in that** inside the separating member (7) one or more flow ducts opening firstly into the reaction chamber (8) and secondly into the reservoir chamber (14) are provided.

13. An apparatus according to one of Claims 5 to 12,
**characterised in that** the flow duct is formed by an annular gap (9) provided between the separating member (7) and the inner wall of the receptacle (3) or an edge profile
and **in that** this flow duct or these flow ducts is/are provided for a height-adjustable separating member (7) at least in the lifting region between the position close to the bottom and the position of the separating member (7) far from the bottom.

14. An apparatus according to one of Claims 1 to 13,
**characterised in that** the under side of the separating member (7) comprise a profiling which diverts gas bubbles to the outside, preferably a convex curvature.

15. An apparatus according to one of Claims 5 to 14,
**characterised in that** a limiting device is provided for the separating member (7)
and **in that** for this purpose a stop which is effective in the position close to the bottom and is preferably adjacent to the upper container edge is provided at the separating member (7).

16. An apparatus according to Claim 15,
**characterised in that** the stop at the separating member (7) which is adjacent to the upper container edge is formed by a cover (13) which overlaps the receptacle edge or by a cover engaging with a cone portion in a complementary cone of the container opening.

17. An apparatus according to one of Claims 1 to 16,
**characterised in that** the separating member (7) comprises at its upper side a standardised opening, preferably a cone for coupling with a pipette, a pipette tip or a dispenser duct.

18. An apparatus according to one of Claims 5 to 17,
**characterised in that** the volume of the reservoir chamber (14, 14a) which is by a multiple greater than the volume of the reaction chamber (8, 8a) is preferably in the ratio of 10:1 to roughly 100:1.

19. An apparatus according to one of Claims 1 to 18,
**characterised in that** at least one chip having one or more microsensors is disposed at least at the bottom of the receptacle (3) or the receiver.

20. An apparatus according to one of Claims 1 to 19,
**characterised in that** sensors (6) and/or electrodes are provided at the separating member (7), facing the reaction chamber (8) and/or the reservoir chamber (14).

21. An apparatus according to one of Claims 5 to 20,
**characterised in that** spaced from the receptacle bottom and between this and the separating member (7) situated in the position close to the bottom is provided a microporous membrane (23) or similar filter or protective cover for the cell culture (2).

22. An apparatus according to one of Claims 1 to 21,
**characterised in that** the separating member (7) is made from smooth, cell-repellent, inert and easily sterilisable material, preferably polytetrafluoroethylene.

23. An apparatus according to one of Claims 1 to 22,
**characterised in that** the bearing surface for the cell culture (2) is optically transparent
and **in that** associated with the bearing surface is an optical measuring instrument, which is preferably disposed at the under side.

24. An apparatus according to one of Claims 5 to 23,
**characterised in that** a plurality of receptacles (3) are provided, preferably as a part of an automatic pipetting device (19),
**in that** these receptacles (3) are formed by commercially available multi-well plates (20)
and **in that** the separating members (7) are provided in each case at the lower ends of the dispenser ducts of the automatic pipetting device.

25. An apparatus according to one of Claims 1 to 24,
**characterised in that** the separating member (7) at its side further from the bottom comprises a connection for connecting, in particular for placing on a dispensing duct (22) preferably of an automatic pipetting device.

## Revendications

1. Dispositif pour effectuer des analyses sur des cellules vivantes, des cultures cellulaires et analogues, en particulier pour détecter l'activité métabolique des cellules qui se trouvent dans un milieu liquide, le dispositif présentant au moins un logement pour le milieu liquide et la culture cellulaire, avec un ou plusieurs dispositifs de mesure et/ou capteurs permettant d'effectuer des mesures prévues au niveau de la culture cellulaire, et avec un corps de séparation mobile prévu pour limiter un compartiment de réaction,
**caractérisé en ce que**
le corps de séparation (7, 7a), dans la position de mesure, est positionné au-dessus et à proximité d'une culture cellulaire située au fond du logement, et sépare le compartiment de réaction (8, 8a) d'un compartiment de réserve, dont le volume de réception est plusieurs fois supérieur au volume de réception du compartiment de réaction (8, 8a), et le corps de séparation (7, 7a) est mobile soit selon un mouvement de va-et-vient pour l'essentiel vertical, entre la position de mesure recouvrant la culture cellulaire et proche du fond et une position éloignée du fond, dans laquelle au moins le compartiment de réserve (14, 14a) est en liaison fluidique avec le compartiment de réaction (8, 8a), soit selon un mouvement de va-et-vient latéral entre la position de mesure et une position dans laquelle le compartiment de réaction (8, 8a) est en liaison fluidique avec le compartiment de réserve (14, 14a).

2. Dispositif selon la revendication 1,
**caractérisé en ce qu'**
au moins un corps de séparation peut être positionné, par rapport à une ou plusieurs culture(s) cellulaire(s) (2) présente(s) au fond du logement, respectivement latéralement afin de déplacer un volume partiel du milieu de culture liquide (4) recouvrant la culture cellulaire et peut être le cas échéant rapproché à cet endroit.

3. Dispositif selon la revendication 1 ou 2,
**caractérisé en ce que**
le ou les capteur(s) (6) est(sont) disposé(s) dans le ou au niveau du fond du logement (3a), des zones de culture séparées les unes des autres, en particulier par des couches déposées de façon structurée et résistantes aux cellules ou par une conception tridimensionnelle du fond comportant des creux ou des bosses, sont formées dans les zones de séparation qui se trouvent dans l'espace intermédiaire, et dans les zones de culture, le milieu de culture (4) est de préférence respectivement présent sous forme de goutte (25).

4. Dispositif selon l'une des revendications 1 à 3,
**caractérisé en ce que**
le fond du logement est formé par au moins une partie d'une plaquette présentant au moins les capteurs (6).

5. Dispositif selon le préambule de la revendication 1,
**caractérisé en ce qu'**
il est prévu en tant que logement un réceptacle (3) ouvert sur le haut, dans lequel se dresse un corps de séparation (7) qui sépare le compartiment de réception global du réceptacle (3) en deux compartiments partiels situés l'un au-dessus de l'autre, le compartiment partiel situé du côté du fond forme un compartiment de réaction (8) à faible volume par rapport au volume de réception global du réceptacle (3) tandis que l'autre compartiment partiel forme un compartiment de réserve (14), dont le volume de réception est plusieurs fois supérieur à celui du compartiment de réaction (8), au moins un canal d'écoulement (9) est d'une part raccordé au compartiment de réaction (8) et d'autre part au compartiment de réserve (14), et à l'intérieur du corps de séparation (7) un ou plusieurs canaux traversants (15) débouchent dans le compartiment de réaction (8) à faible volume du réceptacle (3) et/ou dans le compartiment de réserve (14) du réceptacle.

6. Dispositif selon l'une des revendications 1 à 5,
**caractérisé en ce qu'**
un ou plusieurs capteurs (6) et/ou dispositifs de mesure sont disposés dans la zone du compartiment de réaction (8, 8a).

7. Dispositif selon la revendication 5 ou 6,
**caractérisé en ce que**
le corps de séparation (7) est mobile selon un mouvement de va-et-vient à l'intérieur du réceptacle (3) entre une position proche du fond et une position éloignée du fond.

8. Dispositif selon l'une des revendications 1 à 7,
**caractérisé en ce que**
le côté du corps de séparation (7) tourné vers le fond (5) et servant la mesure de la surface couverte ou limitée par les substances consommées ou formées par les cellules, correspond pour l'essentiel à la surface de détection.

9. Dispositif selon l'une des revendications 5 à 9,
**caractérisé en ce que**
le corps de séparation (7) peut être introduit dans le réceptacle (3) par le haut.

10. Dispositif selon l'une des revendications 1 à 9,
**caractérisé en ce que**
la distance entre le corps de séparation (7) et la culture cellulaire (2), et ainsi la position proche du fond du corps de séparation (7), est réglable.

11. Dispositif selon l'une des revendications 1 à 10,
**caractérisé en ce que**
le corps de séparation (7) est configuré sous forme de tampon et possède une tête (10) dotée d'une section transversale à peu près adaptée à la section transversale du réceptacle (3), qui divise le réceptacle (3) en le compartiment de réaction (8) et le compartiment de réserve (14), un prolongement (11) menant vers l'extérieur est rattaché à la tête du corps de séparation, prolongement dont la section transversale extérieure est inférieure à la section transversale intérieure du réceptacle, et l'espace intermédiaire entre le prolongement et la paroi intérieure du réceptacle (3) forme le compartiment de réserve (4).

12. Dispositif selon l'une des revendications 5 à 11,
**caractérisé en ce qu'**
un ou plusieurs canaux d'écoulement débouchant d'une part dans le compartiment de réaction (8) et d'autre part dans le compartiment de réserve (14) sont prévus à l'intérieur du corps de séparation (7).

13. Dispositif selon l'une des revendications 5 à 12,
**caractérisé en ce que**
le canal d'écoulement est formé par une fente annulaire (9) prévue entre le corps de séparation (7) et la paroi intérieure du réceptacle (3) ou par un profil de bordure, et ce canal d'écoulement ou ces canaux d'écoulement, dans le cas d'un corps de séparation (7) réglable en hauteur, se situe(nt) au moins dans la zone de la course entre la position proche du fond et la position éloignée du fond du corps de séparation (7).

14. Dispositif selon l'une des revendications 1 à 13,
**caractérisé en ce que**
le dessous du corps de séparation (7) présente un profil déviant les bulles de gaz vers l'extérieur, de préférence une courbure convexe.

15. Dispositif selon l'une des revendications 5 à 14,
**caractérisé en ce qu'**
il est prévu une limitation de course pour le corps de séparation (7), et à cet effet une butée au niveau du corps de séparation (7), agit dans la position proche du fond et repose de préférence au niveau du bord supérieur du récipient.

16. Dispositif selon la revendication 15,
**caractérisé en ce que**
la butée reposant au niveau du bord supérieur du récipient au niveau du corps de séparation (7) est formée par un couvercle (13) dépassant sur le bord du récipient ou par un couvercle s'engrenant avec une section en cône dans une partie conique correspondante de l'ouverture du récipient.

17. Dispositif selon l'une des revendications 1 à 16,
**caractérisé en ce que**
le corps de séparation (7) présente sur le dessus une ouverture de réception normalisée, de préférence un cône de réception destiné au raccordement avec une pipette, une pointe de pipette ou un canal de distribution.

18. Dispositif selon l'une des revendications 5 à 17,
**caractérisé en ce que**
le rapport entre le volume de réception du compartiment de réserve (14, 14a), plusieurs fois supérieur au volume de réception du compartiment de réaction (8, 8a), et ce dernier, est de préférence compris entre 10:1 et environ 100:1.

19. Dispositif selon l'une des revendications 1 à 18,
**caractérisé en ce qu'**
au moins une puce comportant un ou plusieurs microcapteurs est disposée au moins au fond du réceptacle (3) ou du logement.

20. Dispositif selon l'une des revendications 1 à 19,
**caractérisé en ce qu'**
il est prévu des capteurs (6) et/ou des électrodes au niveau du corps de séparation (7), tournés vers le compartiment de réaction (8) et/ou vers le compartiment de réserve (14).

21. Dispositif selon l'une des revendications 5 à 20,
**caractérisé en ce qu'**
il est prévu à une certaine distance du fond du réceptacle, entre celui-ci et le corps de séparation (7) se trouvant dans la position proche du fond, une membrane microporeuse (23) ou un filtre ou revêtement de protection analogue pour la culture cellulaire (2).

22. Dispositif selon l'une des revendications 1 à 21,
**caractérisé en ce que**
le corps de séparation (7) se compose d'un matériau lisse, résistant aux cellules, inerte et facile à stériliser, de préférence du polytétrafluoroéthylène.

23. Dispositif selon l'une des revendications 1 à 22,
**caractérisé en ce que**
la surface de séjour de la culture cellulaire (2) est transparente et à la surface de pose est associé un dispositif de mesure optique, qui est de préférence disposé sur le dessous.

24. Dispositif selon l'une des revendications 5 à 23,
**caractérisé en ce qu'**
il est prévu plusieurs réceptacles (3), de préférence en tant que partie d'un pipetteur automatique (19), ces réceptacles (3) sont formés par des plaques multipuits (20) disponibles dans le commerce et les corps de séparation (7) sont respectivement prévus au niveau des extrémités inférieures des canaux de distribution du pipetteur automatique.

25. Dispositif selon l'une des revendications 1 à 24,
**caractérisé en ce que**
le corps de séparation (7) présente, au niveau de son côté opposé au fond, un raccord permettant de raccorder, en particulier de brancher, un canal de distribution (22), de préférence d'un pipetteur automatique.
